(19) Europäisches Patentamt
European Patent Office
Office européen des brevets

(11) Veröffentlichungsnummer: **0 077 501**
**B1**

(12) # EUROPÄISCHE PATENTSCHRIFT

(45) Veröffentlichungstag der Patentschrift: **22.05.85**

(51) Int. Cl.⁴: **C 07 B 39/00**, C 07 C 25/13, C 07 C 17/22, C 07 C 113/04

(21) Anmeldenummer: **82109308.5**

(22) Anmeldetag: **08.10.82**

(54) **Verfahren zur Herstellung von in o-Stellung substituierten Fluoraromaten.**

(30) Priorität: **21.10.81 DE 3141659**

(43) Veröffentlichungstag der Anmeldung: **27.04.83 Patentblatt 83/17**

(45) Bekanntmachung des Hinweises auf die Patenterteilung: **22.05.85 Patentblatt 85/21**

(84) Benannte Vertragsstaaten: **BE CH DE FR GB IT LI NL**

(56) Entgegenhaltungen: **EP - A - 0 022 959**

**JOURNAL OF ORGANIC CHEMISTRY, Band 44, Nr. 9, 1979, Seiten 1572-1574, Easton, USA**

(73) Patentinhaber: **BAYER AG, Konzernverwaltung RP Patentabteilung, D-5090 Leverkusen 1 Bayerwerk (DE)**

(72) Erfinder: **Diehl, Herbert, Dr., Heymannstrasse 40, D-5090 Leverkusen 1 (DE)**
Erfinder: **Pelster, Heinrich, Dr., Auf dem Heidchen 23, D-5068 Odenthal (DE)**
Erfinder: **Habetz, Hubert, Am Weidenbusch 7, D-5090 Leverkusen 3 (DE)**

**EP 0 077 501 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von in o-Stellung substituierten Fluoraromaten aus den entsprechenden aromatischen Aminen.

Es ist bekannt, aromatische Fluorverbindungen mit kerngebundenem Fluor aus den entsprechenden Aminen entweder durch Diazotierung in wasserfreier Flusssäure und anschliessendes Erwärmen der Diazoniumfluoridlösung oder durch Fällung der schwerlöslichen Diazoniumtetrafluoroborate und deren thermische Zersetzung herzustellen (vgl. Houben-Weyl, „Methoden der organischen Chemie", Bd. V/3, S. 213 ff. [1962]).

Während die Diazotierung in wasserfreier Flusssäure zwar gute Ergebnisse bei einfach gebauten aromatischen Aminen liefert, werden nur mässige Ausbeuten bei Aminen erreicht, die in o-Stellung zur Aminogruppe einen Substituenten tragen, der Halogen oder Sauerstoff enthält. So erhält man z.B. gemäss der US-PS Nr. 3950444 2-Bromfluorbenzol in nur maximal 66%iger Ausbeute und 2-Chlorfluorbenzol in nur 28%iger Ausbeute, wenn man 2-Bromanilin bzw. 2-Chloranilin in wasserfreier Flusssäure diazotiert und anschliessend das Diazoniumfluorid thermisch zersetzt.

Dagegen ist die Darstellung von aromatischen Fluorverbindungen über die Diazoniumtetrafluoroborate wesentlich breiter anwendbar. Von Nachteil ist jedoch, dass die Zersetzungsreaktion bei vielen Diazoniumtetrafluoroboraten nur in verhältnismässig kleinen Mengen vorgenommen werden kann. Aus diesem Grunde hat das Verfahren im Gegensatz zu der Diazotierung in wasserfreier Flusssäure noch keinen Eingang in die Technik gefunden (s. Houben-Weyl, „Methoden der organischen Chemie", Bd. V/3, S. 216 [1962]). Ausserdem hat diese Herstellungsweise, bei der die Diazoniumtetrafluoroborate überwiegend im wässerigen System mit Alkalinitrit, Alkylnitrit oder Nitrosylchlorid als Diazotierungsagentien und Tetrafluoroborsäure oder Alkalitetrafluoroborat als Quelle der Tetrafluoroborationen hergestellt werden, noch den Nachteil, dass aufgrund der korrosiven Natur des Reaktionsmediums (wässerige Tetrafluoroborsäure) nur spezielle, aufwendige Apparatewerkstoffe eingesetzt werden können.

Die Herstellung von aromatischen Diazoniumtetrafluoroboraten in nichtwässerigen Systemen durch Umsetzung der entsprechenden Amine mit tert.-Butylnitrit und $BF_3$-Etherat gelingt nach „J. org. Chem.", *44* (9), 1572 (1979) im allgemeinen in guten Ausbeuten, wenn in wasserfreien organischen Lösungsmitteln, wie Tetrahydrofuran, Dimethoxyethan und Diethylether, vorzugsweise Dichlormethan, gearbeitet wird.

Da bei dieser Methode ein grosser Überschuss an $BF_3$-Etherat verwendet wird, ist diese Methode aus wirtschaftlichen Gründen nicht zur Herstellung von aromatischen Diazoniumtetrafluoroboraten in technischem Massstab geeignet. Ausserdem werden nach dieser Methode nur unbefriedigende Ausbeuten an z.B. 3-Brom-4-fluortoluol erhalten, da das entsprechende Diazoniumtetrafluoroborat durch das Ausgangsmaterial, 2-Brom-4-methylaniliniumtetrafluoroborat, verunreinigt ist (s. Beispiel 1a/Vergleichsbeispiel).

Es wurde nun ein Verfahren zur Herstellung von in o-Stellung substituierten Fluoraromaten der Formel:

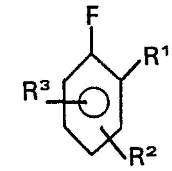

(I)

in der

$R^1$ für Halogen, für die Trihalogenmethyl-, Alkoxy-, Trifluormethoxy-, Carboxyl- oder die Alkoxycarbonylgruppe steht, und

$R^2$ und $R^3$ gleich oder verschieden sind und für Wasserstoff, Halogen, die Alkyl-, Trihalogenmethyl-, Alkoxy-, Trifluormethoxy-, Carboxyl- oder die Alkoxycarbonylgruppe stehen,

gefunden, das dadurch gekennzeichnet ist, dass man in o-Stellung substituierte Aniline der Formel:

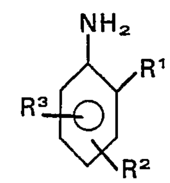

(II)

in der

$R^1$, $R^2$ und $R^3$ die obengenannte Bedeutung haben, mit Alkylnitriten in Gegenwart von Alkoholen und/oder Ethern, gegebenenfalls unter Zusatz eines inerten organischen Verdünnungsmittels, und in Anwesenheit von 1,0 bis 2,0 mol Bortrifluorid oder Borsäureester und Fluorwasserstoff pro Mol o-substituiertem Anilin bei Temperaturen von −20 bis +10°C umsetzt, das entstehende Diazoniumtetrafluoroborat isoliert und dieses gegebenenfalls nach Trocknung in Gegenwart eines inerten Wärmeträgermediums bei erhöhter Temperatur zersetzt.

Als Halogene seien genannt: Fluor, Chlor, Brom und Jod, bevorzugt Fluor, Chlor und Brom, besonders bevorzugt Brom; als Alkylreste solche mit 1 bis 10, bevorzugt 1 bis 4 Kohlenstoffatomen, wie der Methyl-, Ethyl-, n-Propyl-, Isopropyl-, n-Butyl-, Isobutyl-, tert.-Butyl-, n-Pentyl-, Isopentyl-, Hexyl-, Heptyl-, Octyl-, Nonyl- und der Decylrest, bevorzugt der Methyl-, Ethyl-, n-Propyl- und der n-Butylrest; als Trihalogenmethylreste der Trifluormethyl- und der Trichlormethylrest, bevorzugt der Trifluormethylrest; als Alkoxyreste solche mit 1 bis 10, bevorzugt 1 bis 3 Kohlenstoffatomen, wie der Methoxy-, Ethoxy-, Propoxy-, Butoxy- und der Pentoxyrest, bevorzugt der Methoxy-, Ethoxy- und der Propoxyrest, und als Alkoxycarbonylreste solche mit 1 bis 10, bevorzugt 1 bis 5 Kohlenstoffatomen, wie der Methoxycarbonyl-, Ethoxycarbonyl-, Carboxycarbonyl-, Butoxycarbonyl-, Pentoxycarbonyl- und der Hexoxycarbonylrest, bevorzugt der Methoxycarbonyl-, Ethoxycarbonyl- und der Propoxycarbonylrest.

In das erfindungsgemässe Verfahren können als o-substituierte Aniline der Formel (II) eingesetzt werden:

2-Bromanilin, 2-Methoxyanilin, 2-Ethoxycarbonylanilin, 2-Trifluormethyl-4-bromanilin, 2-Chloranilin, 2-Brom-4-methylanilin, 2,4-Dichlor-5-aminotoluol und 2-Brom-4,6-dimethylanilin.

Bevorzugt werden eingesetzt: 2-Brom-4-methylanilin, 2-Brom-4,6-dimethylanilin und 2-Chloranilin.

Als Alkylnitrite können solche mit 1 bis 10, bevorzugt 1 bis 5 Kohlenstoffatomen, wie Methylnitrit, Ethylnitrit, Isopropylnitrit, Isobutylnitrit, Isoamylnitrit, Isohexylnitrit und Isoheptylnitrit, bevorzugt Methylnitrit, Isopropylnitrit und Isobutylnitrit, in das erfindungsgemässe Verfahren eingesetzt werden.

Die Menge der einzusetzenden Alkylnitrite ist nicht kritisch. Im allgemeinen werden etwa 1,0 bis 2,0, bevorzugt 1,05 bis 1,15 mol Alkylnitrit pro Mol aromatische Aminoverbindung eingesetzt.

Als Alkohole können solche mit 1 bis 10, bevorzugt 1 bis 5 Kohlenstoffatomen, wie Methanol, Ethanol, n-Propanol, Isopropanol, n-Butanol, Isobutanol, tert.-Butanol, n-Pentanol, Isopentanol, n-Hexanol und n-Heptanol, bevorzugt Methanol, Ethanol, Isopropanol und Isobutanol, in das erfindungsgemässe Verfahren eingesetzt werden.

Als Ether können solche mit 4 bis 10, bevorzugt 4 bis 8 Kohlenstoffatomen, wie Tetrahydrofuran, Dimethoxyethan und Diethoxyethan, bevorzugt Tetrahydrofuran und Dimethoxyethan, in das erfindungsgemässe Verfahren eingesetzt werden.

Die Alkohole und die Ether können sowohl einzeln als auch im Gemisch untereinander in das erfindungsgemässe Verfahren eingesetzt werden, wobei die Alkohole und die Ether in jedem beliebigen Verhältnis miteinander vermischt sein können.

Die Menge der einzusetzenden Alkohole und/oder Ether kann in weiten Bereichen schwanken. Üblicherweise werden 200 bis 1000, bevorzugt 250 bis 500, bezogen auf 100 g Anilinderivat, eingesetzt.

Das erfindungsgemässe Verfahren kann gegebenenfalls unter Zusatz eines inerten organischen Verdünnungsmittels, wie Dichlormethan, Chlorbenzol und/oder Toluol, bevorzugt Dichlormethan, durchgeführt werden. Dabei wird im allgemeinen 100 bis 1000 g, bezogen auf 100 g Anilinderivat, an inertem organischem Verdünnungsmittel dem eingesetzten Alkohol und/oder Ether zugegeben.

Die Menge an Bortrifluorid und Fluorwasserstoff, die in das erfindungsgemässe Verfahren eingesetzt wird, beträgt 1,0 bis 2,0, bevorzugt 1,05 bis 1,50 mol Bortrifluorid und Fluorwasserstoff pro Mol aromatisches Amin. Zweckmässigerweise arbeitet man mit einem molaren Überschuss an Bortrifluorid und Fluorwasserstoff, bezogen auf eingesetztes aromatisches Amin.

Das molare Verhältnis von Bortrifluorid zu Fluorwasserstoff kann in weiten Bereichen schwanken. Allerdings ist es vorteilhaft, den Fluorwasserstoff im geringen Unterschuss gegenüber dem Bortrifluorid einzusetzen. So wird im allgemeinen, 1,01 bis 1,50, bevorzugt 1,05 bis 1,20 mol Bortrifluorid pro Mol Fluorwasserstoff verwendet.

Nach einer anderen Variante des erfindungsgemässen Verfahrens kann die Umsetzung statt mit Bortrifluorid und Fluorwasserstoff auch mit Borsäureestern, wie Trimethyl-, Triethyl-, Triisopropyl- und/oder Tributylborat, bevorzugt Triisopropylborat und/oder Triisobutylborat, und Fluorwasserstoff durchgeführt werden. Dabei werden die Borsäureester und der Fluorwasserstoff in dem entsprechenden molaren Verhältnis untereinander und in einer Menge wie das Bortrifluorid und Fluorwasserstoff in das erfindungsgemässe Verfahren eingesetzt.

Die erfindungsgemässe Diazotierung wird üblicherweise bei Temperaturen von ca. −20 bis +20, bevorzugt bis +5° C, durchgeführt.

Das bei der erfindungsgemässen Umsetzung gebildete Diazoniumtetrafluoroborat wird in üblicher Weise isoliert, z.B. durch Filtrieren oder Zentrifugieren, und gegebenenfalls nach Trocknung in Gegenwart eines Wärmeträgermediums thermisch zersetzt.

Als Wärmeträgermedien dienen z.B. halogenierte Aromaten, wie Chlorbenzol, Dichlorbenzole und/oder 1-Chlornaphthalin sowie Paraffinöl und/oder alkylierte Aromaten, wie Xylol und Triisopropylbenzol.

Bevorzugt werden als Wärmeträgermedium Paraffinöl und Triisopropylbenzol verwendet.

Die thermische Zersetzung der Diazoniumtetrafluoroborate wird zweckmässigerweise bei einer Temperatur durchgeführt, die bis zu etwa 50°C über der Zersetzungstemperatur des jeweiligen Diazoniumtetrafluoroborats liegt. Im allgemeinen wird die Zersetzung bei etwa 100 bis 200, bevorzugt 100 bis 150° C, durchgeführt.

Das bei der Zersetzung freiwerdende Bortrifluorid kann nach einer bevorzugten Arbeitsweise in einem oder mehreren der zuvor erwähnten Alkohole und/oder Ether absorbiert und auf diese Weise in die Reaktion zurückgeführt werden.

Das erfindungsgemässe Verfahren kann in der Weise durchgeführt werden, dass man zu dem vorgelegten, Bortrifluorid oder Borsäureester und Fluorwasserstoff enthaltenden Alkohol und/oder Ether zunächst das aromatische Amin und dann das Alkylnitrit oder umgekehrt zuerst das Alkylnitrit und danach das aromatische Amin gibt. Weiterhin ist es möglich, den Alkohol und/oder Ether zusammen mit Bortrifluorid oder Borsäureester und dem aromatischen Amin vorzulegen und zum Schluss den Fluorwasserstoff und Alkylnitrit zuzudosieren. Besonders vorteilhaft erwies sich jedoch die synchrone Dosierung des aromatischen Amins und des Alkylnitrits in den vorgelegten, Bortrifluorid und Fluorwasserstoff oder Borsäureester und Fluorwasserstoff enthaltenden Alkohol und/oder Ether.

Man kühlt das Reaktionsgemisch auf etwa −10 bis 0° C ab und filtriert nach Ende der Reaktion das aus dem Lösungsmittel ausfallende Diazoniumtetrafluoroborat ab. Nach dem Waschen des Diazoniumtetrafluoroborats mit beispielsweise einem Isobutanol/Methanol-Gemisch oder einem inerten Lösungsmittel wird gegebenenfalls im Vakuum getrocknet und anschliessend das Diazoniumtetrafluoroborat durch Eintragen in z.B. vorgelegtes Paraffinöl, das auf etwa 50°C über der Zersetzungstemperatur des entsprechenden Diazoniumtetrafluoroborats aufgeheizt wird, zersetzt. Nach der Zersetzung des Diazoniumtetrafluoroborats wird die aromatische Fluorverbindung im Vakuum abdestilliert, das Destillat mit einer basisch reagierenden Verbindung, z.B. Calciumoxid, behandelt, die basische Verbindung abfiltriert und die aromatische Fluorverbindung durch erneute Destillation isoliert.

Das bei der Zersetzung des Diazoniumtetrafluoroborats freiwerdende Bortrifluorid wird zweckmässigerweise in dem bei der Umsetzung eingesetzten Alkohol und/oder Ether absorbiert und in die Reaktion zurückgeführt.

Nach dem erfindungsgemässen Verfahren werden in o-Stellung substituierte Fluoraromaten in guten Ausbeuten (bis 90% der Theorie) und hohen Reinheiten ($\geqslant$ 95%) erhalten. Dies ist besonders überraschend, da es bislang technisch nur in unbefriedigendem Masse möglich war, die Aminogruppe gegen Fluor über eine Diazoniumverbindung auszutauschen, wenn in o-Stellung zur Aminogruppe ein Substituent vorhanden war, der Halogen oder Sauerstoff enthielt (vgl. Houben-Weyl, „Methoden der organischen Chemie", Bd. V/3, S. 214, 216 [1962]).

Die nach dem erfindungsgemässen Verfahren hergestellten o-substituierten Fluoraromaten sind wertvolle Ausgangsstoffe bei der Herstellung von Wirkstoffen.

So dient das 3-Brom-4-fluortoluol als Vorstufe bei der Herstellung von 4-Fluor-3-phenoxytoluol (DE-OS Nrn. 2837525, 2932093), welches seinerseits als Grundstoff bei der Herstellung von Insektiziden eingesetzt wird (DE-OS Nrn. 2837524, 2844816; EP Nr. 9708).

Die nachfolgenden Beispiele sollen das erfindungsgemässe Verfahren verdeutlichen, ohne es jedoch auf diese Beispiele einzuschränken.

*Beispiel 1:*

(Vergleichsbeispiel durchgeführt nach „J. org. Chem.", *44* [9], 1572 [1979])

a) Dichlormethan

Zu 105,4 g Bortrifluoridetherat tropft man bei −15°C eine Lösung von 92 g 2-Brom-4-methylanilin in 500 ml Dichlormethan. Man rührt 30 min bei dieser Temperatur nach und lässt dann auf +5°C ansteigen.

Man tropft nun eine Lösung von 81 g tert.-Butylnitrit in 250 ml Dichlormethan bei +5°C zu (innerhalb 1 h). Man rührt noch 15 min bei +5°C nach und saugt das ausgefallene Salz ab. Der Feststoff wird mit ca. 200 ml Dichlormethan gewaschen und bei Raumtemperatur im Vakuum getrocknet. Man erhält 127 g eines kristallinen Feststoffs, der 90 bis 95% 2-Brom-4-methylbenzoldiazoniumtetrafluoroborat enthält, 81 bis 85% der Theorie (Rest: 2-Brom-4-methylaniliniumtetrafluoroborat).

Die Zersetzung gemäss Beispiel 6 liefert neben hohen Anteilen an harzigen Nebenprodukten 53 g 3-Brom-4-fluortoluol (Ausbeute: 56,6% der Theorie über beide Stufen).

b) Tetrahydrofuran

Zu einer Lösung von 51 g Bortrifluorid in 200 ml Tetrahydrofuran tropft man bei −15°C 93 g 2-Brom-4-methylanilin. Man rührt 30 min nach und tropft dann bei 0 bis 5°C 62 g tert.-Butylnitrit zu. Man rührt 1 h bei 0°C nach und filtriert den Rückstand ab.

Der Filterkuchen wird mit ca. 10 ml kaltem Tetrahydrofuran gewaschen und danach im Vakuum bei Raumtemperatur getrocknet.

Man erhält 112 g 2-Brom-4-methylbenzoldiazoniumtetrafluoroborat (78,6% der Theorie).

Die Zersetzung gemäss Beispiel 6 liefert 63 g 3-Brom-4-fluortoluol (85% der Theorie).

Die Ausbeute über beide Stufen beträgt 66,8% der Theorie.

*Beispiel 2:*

Zu einer Lösung von 71 g Bortrifluorid und 20,4 g Fluorwasserstoff in 315 g Isopropanol tropft man bei 0 bis 5°C 186 g 2-Brom-4-methylanilin und leitet gleichzeitig 67,0 g Methylnitrit ein.

Man rührt anschliessend noch 1 h bei 0°C nach und filtriert das ausgefallene Diazoniumsalz ab. Der Feststoff wird mit insgesamt 120 g Isopropanol gewaschen und danach bei Raumtemperatur im Vakuum getrocknet.

Man erhält auf diese Weise 275 g (96,5% der Theorie) 2-Brom-4-methylbenzoldiazoniumtetrafluoroborat.

Die Zersetzung nach Beispiel 6 liefert 3-Brom-4-fluortoluol in einer Ausbeute von 92,4% der Theorie.

*Beispiel 3:*

Zu einer Lösung von 30 g Fluorwasserstoff in 150 g Isopropanol tropft man bei 0°C unter Kühlung zunächst 71 g Borsäuretripropylester und danach 67,5 g 2-Brom-4-methylanilin. Man rührt 30 min bei 0°C nach und tropft dann bei −5 bis 0°C innerhalb von 15 min 36 g Isopropylnitrit zu. Man rührt weitere 15 min nach, saugt ab und wäscht den Rückstand mit wenig kaltem Isopropanol.

Nach Trocknen im Vakuum bei Raumtemperatur erhält man 98,5 g 2-Brom-4-methylbenzoldiazoniumtetrafluoroborat (96% der Theorie).

Die Zersetzung gemäss Beispiel 6 liefert das 3-Brom-4-fluortoluol in einer Ausbeute von 92,4% der Theorie.

*Beispiel 4:*

Zu einer Lösung von 37 g Bortrifluorid und 10,3 g Fluorwasserstoff in 160 g Dimethoxyethan tropft man bei −5 bis 0°C simultan aus 2 Tropftrichtern 84 g 2-Brom-4-methylanilin und 54,5 g Isobutylnitrit (Dauer 1 h). Man rührt noch 15 min bei 0°C nach und saugt dann ab. Das kristalline Diazoniumsalz wird mit 50 ml kaltem Dimethoxyethan gewaschen und danach bei Raumtemperatur im Vakuum getrocknet.

Ausbeute: 122,5 g (86% der Theorie).

Die Zersetzung gemäss Beispiel 6 liefert 3-Brom-4-fluortoluol in einer Ausbeute von 92,4% der Theorie.

*Beispiel 5:*

Zu einer Lösung von 71 g Bortrifluorid und 20,4 g Fluorwasserstoff in 400 g Isobutanol tropft man bei 0 bis 5°C simultan aus zwei Tropftrichtern 186 g 2-Brom-4-methylanilin und 122 g Isobutylnitrit (93,5%ig in Isobutanol) (Dauer ca. 3 h).

Man rührt 1 h bei 0°C nach, filtriert ab und wäscht den Feststoff mit 150 g Isobutanol nach.

Nach Trocknung im Vakuum bei Raumtemperatur erhält man 280 g 2-Brom-4-methylbenzoldiazoniumtetrafluoroborat (98,2% der Theorie).

Die Zersetzung nach Beispiel 6 liefert 3-Brom-4-fluortoluol in einer Ausbeute von 92,4% der Theorie.

*Beispiel 6:*

Im Verlauf von 5 h werden 143 g 2-Brom-4-methylbenzoldiazoniumtetrafluoroborat (hergestellt gemäss den Beispielen 2 bis 5) bei 120 bis 125°C auf 80 g Paraffinöl eindosiert.

Das Abgas (Bortrifluorid und Stickstoff) wird durch einen Wäscher geschickt, der bei 0°C mit 100,0 g Isobutanol betrieben wird.

Die Reaktionsmischung wird nach beendeter Dosierung noch etwa 1 h bei 125 bis 130°C nachgerührt.

Anschliessend werden flüchtige Bestandteile im Vakuum abdestilliert.

Das Destillat wird mit 1 g Calciumoxid versetzt und filtriert. Man erhält auf diese Weise 88,2 g 3-Brom-4-fluortoluol von 99%iger Reinheit (Ausbeute: 92,4% der Theorie).

Der Inhalt des Abgaswäschers (25,2% $BF_3$ in Isobutanol, 130 g) kann nach Einstellen der erforderlichen Konzentration zur Herstellung von 2-Brom-4-methylbenzoldiazoniumtetrafluoroborat gemäss Beispiel 5 eingesetzt werden.

Die Rückführrate wurde zu 96,5% der Theorie ermittelt.

*Beispiel 7:*

Im Verlauf von 10 h werden 855 g 2-Brom-4-methylbenzoldiazoniumtetrafluoroborat bei 125 bis 130°C auf 85 g 1,3,5-Triisopropylbenzol eindosiert.

Nach beendeter Dosierung wird noch 1 h auf 125 bis 130°C gehalten (Ende der Gasentwicklung). Anschliessend werden flüchtige Bestandteile im Vakuum abdestilliert. Das Destillat wird mit Calciumoxid neutralisiert und durch Rektifikation gereinigt. Man erhält 474,5 g 3-Brom-4-fluortoluol (98,8%ig), d.h. 82,7% der Theorie.

Der Destillationsrückstand (1,3,5-Triisopropylbenzol, 88,4 g) kann rückgeführt werden.

Das bei der Zersetzung entstehende $BF_3$ wird in Alkohol absorbiert und kann so zwanglos in den Prozess zurückgeführt werden (Ausbeute: > 90% der Theorie).

*Beispiel 8:*

Im Verlauf von 3 h werden 145 g 2-Brom-4-methylbenzoldiazoniumtetrafluoroborat auf 100 g 1-Chlornaphthalin bei 125 bis 130°C eindosiert.

Nach beendeter Dosierung wird noch 1 h bei 125 bis 130°C nachgerührt.

Anschliessend wird unter vermindertem Druck das entstandene 3-Brom-4-fluortoluol abdestilliert. Man erhält nach Neutralisation mit Calciumoxid 72,5 g 3-Brom-4-fluortoluol (76,4% der Theorie).

Das entstandene Bortrifluorid wird in Isopropanol absorbiert (>90% der Theorie) und kann so zur Herstellung des Ausgangsmaterials wiederverwendet werden (s. Beispiel 2).

Der Rückstand (1-Chlornaphthalin und harzige Nebenprodukte) kann nach Neutralisation mit Calciumoxid und Destillation rückgeführt werden.

*Beispiel 9:*

Gemäss Beispiel 5 wurden die folgenden Diazoniumfluoroborate hergestellt:

| Benzoldiazoniumtetrafluoroborat-derivat | Ausbeute (% d. Th.) |
|---|---|
| 2-Brom- | 97,4 |
| 2-Chlor- | 96,7 |
| 2-Brom-4,6-dimethyl- | 99,0 |
| 2-Methoxy- | 92,3 |
| 2-Ethoxycarbonyl- | 97,7 |
| 2-Trifluormethoxy | 97,1 |
| 2-Trifluormethyl-4-brom | 93,5 |

*Beispiel 10:*

Gemäss Beispiel 6 wurden die folgenden Fluorbenzolderivate hergestellt:

| Fluorbenzolderivat | Ausbeute (% d. Th.) |
|---|---|
| 2-Brom- | 84,1 |
| 2-Chlor- | 81,2 |
| 2-Brom-4,6-dimethyl- | 80,5 |
| 2-Methoxy- | 58,7 |
| 2-Ethoxycarbonyl | 65,7 |
| 2-Trifluormethyl-4-brom | 88,5 |

*Beispiel 11:*

Zu einer Lösung von 13 g Fluorwasserstoff und 44 g Bortrifluorid in 275 g Isopropanol werden bei 0 bis +5°C 88 g 2,4-Dichlor-5-aminotoluol in 330 g Toluol und 50 g Methylnitrit zugetropft bzw. eingeleitet.

Nach beendeter Dosierung wird noch 30 min bei 0°C nachgerührt.

Der Rückstand wird dann abgesaugt, mit kaltem Isopropanol (100 g) gewaschen und anschliessend im Vakuum bei Raumtemperatur getrocknet.

Ausbeute: 128,5 g 2,4-Dichlor-5-methylbenzoldiazoniumtetrafluoroborat (93,5% d. Th.).

*Beispiel 12:*

Zu 20 g 2,4-Dichlor-5-fluortoluol werden bei 160-165°C im Verlauf von ca. 3 h 50 g 2,4-Dichlor-5-methylbenzoldiazoniumtetrafluoroborat in kleinen Portionen zudosiert. Nach beendeter Gasentwicklung wird der Rückstand unter vermindertem Druck destilliert.

Man erhält nach Neutralisation mit Calciumoxid 28,5 g 2,4-Dichlor-5-fluortoluol (97% d. Th.).

Das entstandene Bortrifluorid wird in Isopropanol absorbiert (> 90% d. Th.) und kann so zur

Herstellung des Ausgangsmaterials wiederverwendet werden (s. Beispiel 11).

## Patentansprüche

1. Verfahren zur Herstellung von in o-Stellung substituierten Fluoraromaten der Formel:

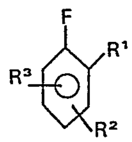

in der

$R^1$ für Halogen, für die Trihalogenmethyl-, Alkoxy-, Trifluormethoxy-, Carboxyl- oder die Alkoxycarbonylgruppe steht, und

$R^2$ und $R^3$ gleich oder verschieden sind und für Wasserstoff, Halogen, die Alkyl-, Trihalogenmethyl-, Alkoxy-, Trifluormethoxy-, Carboxyl- oder die Alkoxycarbonylgruppe stehen,

dadurch gekennzeichnet, dass man in o-Stellung substituierte Aniline der Formel:

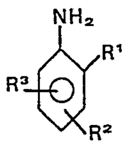

in der

$R^1$, $R^2$ und $R^3$ die obengenannte Bedeutung haben,

mit Alkylnitriten in Gegenwart von Alkoholen und/oder Ethern, gegebenenfalls unter Zusatz eines inerten organischen Verdünnungsmittels, und in Anwesenheit von 1,0 bis 2,0 mol Bortrifluorid oder Borsäureester und Fluorwasserstoff pro Mol o-substituiertem Anilin bei Temperaturen von −20 bis +10°C umsetzt, das entstehende Diazoniumtetrafluoroborat isoliert und dieses gegebenenfalls nach Trocknung in Gegenwart eines inerten Wärmeträgermediums bei erhöhter Temperatur zersetzt.

2. Verfahren nach Anspruch 1, dadurch gekennzeichnet, dass man das bei der Zersetzung freiwerdende Bortrifluorid in einem Alkohol und/oder Ether absorbiert und in die Reaktion zurückführt.

3. Verfahren nach den Ansprüchen 1 und 2, dadurch gekennzeichnet, dass man als o-substituierte Aniline 2-Bromanilin, 2-Methoxyanilin, 2-Ethoxycarbonylanilin, 2-Trifluormethyl-4-bromanilin, 2-Chloranilin, 2-Brom-4-methylanilin, 2,4-Dichlor-5-aminotoluol und 2-Brom-4,6-dimethylanilin einsetzt.

4. Verfahren nach den Ansprüchen 1 bis 3, dadurch gekennzeichnet, dass man 1,0 bis 2,0 mol Alkylnitrit pro Mol o-substituiertem Anilin einsetzt.

5. Verfahren nach den Ansprüchen 1 bis 4, dadurch gekennzeichnet, dass man die Alkohole und/oder die Ether in Mengen von 200 bis 1000 g, bezogen auf 100 g o-substituiertes Anilin, einsetzt.

6. Verfahren nach den Ansprüchen 1 bis 5, dadurch gekennzeichnet, dass man 1,01 bis 1,50 mol Bortrifluorid oder Borsäureester pro Mol Fluorwasserstoff einsetzt.

7. Verfahren nach den Ansprüchen 1 bis 6, dadurch gekennzeichnet, dass man als Wärmeträgermedium halogenierte Aromaten, Paraffinöl und/oder alkylierte Aromaten einsetzt.

8. Verfahren nach den Ansprüchen 1 bis 7, dadurch gekennzeichnet, dass man die thermische Zersetzung des Diazoniumtetrafluoroborats bei Temperaturen von 100 bis 200°C durchführt.

## Claims

1. Process for the preparation of fluoroaromatic compounds substituted in the o-position of the formula:

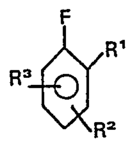

in which

$R^1$ represents halogen, or represents a trihalogenomethyl, alkoxy, trifluoromethoxy, carboxyl or an alkoxycarbonyl group, and

$R^2$ and $R^3$ are identical or different and represent hydrogen, halogen, an alkyl, trihalogenomethyl, alkoxy, trifluoromethoxy, carboxyl or the alkoxycarbonyl group,

characterised in that anilines substituted in the o-position of the formula:

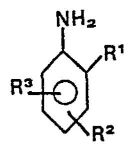

in which

$R^1$, $R^2$ and $R^3$ have the above-mentioned meaning,

are reacted with alkyl nitrites in the presence of alcohols and/or ethers, optionally with the addition of an inert organic diluent, and in the presence of 1.0 to 2.0 mol of boron trifluoride or boric acid ester and hydrogen fluoride per mol of o-substituted aniline at temperatures from −20 to +10°C, the diazonium tetrafluoroborate produced is isolated and the latter, optionally after drying, is decomposed in the presence of an inert heat-transfer medium at an elevated temperature.

2. Process according to Claim 1, characterised in that the borom trifluoride liberated in the decomposition is absorbed in an alcohol and/or ether and is returned to the reaction.

3. Process according to Claims 1 and 2, characterised in that the o-substituted anilines used are 2-bromoaniline, 2-methoxyaniline, 2-ethoxycarbonylaniline, 2-trifluoromethyl-4-bromoaniline, 2-chloroaniline, 2-bromo-4-methylaniline, 2,4-dichloro-5-aminotoluene or 2-bromo-4,6-dimethylaniline.

4. Process according to Claims 1 to 3, characterised in that 1.0 to 2.0 mol of alkyl nitrite are used per mol of o-substituted aniline.

5. Process according to Claims 1 to 4, characterised in that the alcohols and/or the ethers are

used in amounts of 200 to 1,000 g relative to 100 g of o-substituted aniline.

6. Process according to Claims 1 to 5, characterised in that 1.01 to 1,50 mol of boron trifluoride or boric acid ester are used per mol of hydrogen fluoride.

7. Process according to Claims 1 to 6, characterised in that the heat-transfer medium used is halogenated aromatic compounds, paraffin oil and/or alkylated aromatic compounds.

8. Process according to Claims 1 to 7, characterised in that the thermal decomposition of the diazonium tetrafluoroborate is carried out at temperatures from 100 to 200° C.

## Revendications

1. Procédé de préparation d'hydrocarbures aromatiques fluorés substitués en position o et répondant à la formule:

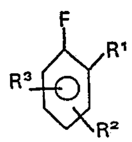

dans laquelle

$R^1$ représente un atome d'halogène, un groupe triahlogénométhyle, un groupe alcoxy, un groupe trifluorométhoxy, un groupe carboxy ou un groupe alcoxycarbonyle, et

$R^2$ et $R^3$, qui sont identiques ou différents, représentent chacun un atome d'hydrogène, un atome d'halogène, un groupe alkyle, un groupe trihalogénométhyle, un groupe alcoxy, un groupe trifluorométhoxy, un groupe carboxy ou un groupe alcoxycarbonyle,

caractérisé en ce qu'on fait réagir des anilines substituées en position o de formule:

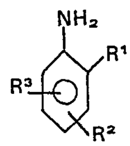

dans laquelle

$R^1$, $R^2$ et $R^3$ ont les significations indiquées ci-dessus,

avec des nitrites d'alkyle en présence d'alcools et/ou d'éthers, en ajoutant éventuellement un diluant organique inerte, ainsi qu'en présence de 1 à 2 mol de trifluorure de bore ou d'un ester d'acide borique et d'acide fluorhydrique par mole d'aniline o-substituée, à des températures de −20 à +10° C, en ce qu'on isole le tétrafluoroborate de diazonium formé et en ce qu'éventuellement après séchage on décompose ce dernier à température élevée en présence d'un milieu caloporteur inerte.

2. Procédé suivant la revendication 1, caractérisé en ce qu'on absorbe le trifluorure de bore libéré lors de la réaction dans un alcool et/ou un éther et en ce qu'on le recycle à la réaction.

3. Procédé suivant les revendications 1 et 2, caractérisé en ce que, comme anilines o-substituées, on utilise la 2-bromaniline, la 2-méthoxyaniline, la 2-éthoxycarbonylaniline, la 2-trifluorométhyl-4-bromaniline, la 2-chloraniline, la 2-bromo-4-méthylaniline, le 2,4-dichloro-5-aminotoluène et la 2-bromo-4,6-diméthylaniline.

4. Procédé suivant les revedications 1 à 3, caractérisé en ce qu'on utilise 1 à 2 mol d'un nitrite d'alkyle par mole d'aniline o-substituée.

5. Procédé suivant les revendications 1 à 4, caractérisé en ce qu'on utilise les alcools et/ou les éthers en quantités de 200 à 1000 g, calculé sur 100 g d'aniline o-substituée.

6. Procédé suivant les revendications 1 à 5, caractérisé en ce qu'on utilise 1,01 à 1,5 mol de trifluorure de bore ou d'un ester d'acide borique par mole d'acide fluorhydrique.

7. Procédé suivant les revendications 1 à 6, caractérisé en ce que, comme milieu caloporteur, on utilise des hydrocarbures aromatiques halogénés, de l'huile paraffinique et/ou des hydrocarbures aromatiques alkylés.

8. Procédé suivant les revendications 1 à 7, caractérisé en ce qu'on effectue la décomposition thermique du tétrafluoroborate de diazonium à des températures de 100 à 200° C.